# EUROPEAN PATENT APPLICATION

(11) **EP 2 532 251 A1**
(43) Date of publication of application: **12.12.2012**
(21) Application number: 12004136.3
(22) Date of filing: 29.05.2012
(51) Int. Cl.: A23L 1/29, A23L 1/30, A61K 31/12, A61K 31/21, C07C 403/00, A61K 36/81, A61P 3/04

(54) **Functional food and pharmaceutical compositions for anti-obesity comprising capsanthin and its fatty acylester derivatives having anti-adipogenic activity**

(30) Priority: 08.06.2011 KR 20110055137; 23.05.2012 KR 20120054734
(71) Applicant: Es Biotech Co., Ltd., Byeongcheon-myeon Dongnam-gu, Cheoan-si Ch'ungch'ong namdo 330-863 (KR)
(72) Inventor: Han, Byung-Hoon, Cheonan-si Chungcheongnam-do 331-738 (KR); Kim, Jeong-Won, Cheonan-si Chungcheongnam-do 330-741 (KR); Jo, Sung-Jun, Cheongwon-gun Chungcheongbuk-do 363-911 (KR); Choi, Hye-Ok, Asan-si Chungcheongnam-do 336-727 (KR); Kim, Jung-Hwan, Cheongju-si Chungcheongbuk-do 360-081 (KR); Kim, Moo-Kang, Daejeon 305-358 (KR); Park, II-Kwon, Seo-gu Daejeon 302-318 (KR); Lee, Seung-Hwan, Nonsan-si Chungcheongnam-do 320-030 (KR)
(74) Representative: Laufhütte, Dieter

(57) **Abstract**

One or more embodiments of the present invention describe a novel use of capsanthin and/or fatty-acyl ester of capsanthin to inhibit both the differentiation of pre-adipocytes to adipocytes and the accumulation of fat in the adipocytes. One or more embodiments of the present invention are based on findings that capsanthin contained in some natural products such as red pepper, paprika, bell pepper, etc. have anti-adipogenic activity by inhibiting both the differentiation of pre-adipocyte into adipocyte and the accumulation of fat in adipocytes, Therefore, this findings provide a functional food and a pharmaceutical composition wherein capsanthins and the extract of any natural products containing the same components are included in the functional food and the pharmaceutical composition, to offer obesity prevention and/or treatment effects.

## Description

### CROSS-REFERENCE TO RELATED APPLICATION AND CLAIM OF PRIORITY

This application claims priority to Korean Patent Application Nos. 10-2011-0055137, filed on June 8, 2011, and 10-2012-0054734, filed on May 23, 2012, which are incorporated herein by reference in their entirety.

### BACKGROUND

1. Field of the Invention

The present invention relates to novel efficacy of capsanthin and fatty-acyl ester of capsanthin to inhibit both the differentiation of pre-adipocytes to adipocytes and the accumulation of triglycerides in the adipocytes.

2. Description of the Related Art

Obesity is arisen due to excessive accumulation of fat in adipocytes, and it is no longer considered simply as a cosmetic problem, but it becomes now as serious health threatening issues concerned with a number of metabolic disorders including diabetes, hypertension, breast cancer, colon cancer, cardiovascular disease, cerebrovascular disease, etc.

It has been reported that obese people have 10-fold and 4-fold increased risks of developing to diabetes and hypertension respectively, compared to those who are not obese.

It is estimated that 40 to 50% of the overall populations in western countries are over-weighted or obese. In Korea, as dietary lives are becoming more westernized, obesity is remaining as the health problem not only in adults but also even in children, and it shows a trend of rapid increase in the prevalence of childhood obesity. Therefore, there is now an urgent need for establishing some strategies for the prevention of obesity and improvement thereof.

Capsaicin, a minor pungent principle of red pepper, has already been described as a potent anti-adipogenic substance by showing the inhibitory activity on the differentiation of pre-adipocytes (3T3-L1 cell) to adipocyte. Chemical structure of capsaicin has been established as an acid-amide type component composed of isodecylenic acid and vanillylamine-moiety.

Meanwhile, 'capsanthin', the carotenoid red pigment in pungent peppers, paprika, bell peppers and so forth, is known to have antioxidant activity, however, no previous scientific papers or patented claims have described for the above anti-adipogenic activity.

### SUMMARY

An aspect of the present invention is to provide an experimental method to demonstrate the inhibitory activities of capsanthin and fatty-acyl ester of capsanthin on the adipogenesis of 3T3-L1 cell.

Another aspect of the present invention is to provide an anti-adipogenic functional food, containing capsanthin or fatty-acyl ester of capsanthin.

A further aspect of the present invention is to provide a pharmaceutical composition for the treatment or prevention of obesity, containing capsanthin or fatty-acyl ester of capsanthin .

In order to accomplish one or more of the above aspects, one or more embodiments of the present invention provide a functional food composition with anti-obesity activity, which includes capsanthin or fatty-acyl ester of capsanthin, or an extract of any natural products containing the same.

The present invention also provides a pharmaceutical composition for treatment or prevention of obesity, which includes capsanthin or fatty-acyl ester of capsanthin, or an extract of any natural products containing the same.

In addition, the present invention provides a method for inhibiting both the differentiation of pre-adipocyte to adipocyte and the accumulation of triglyceride in the adipocytes under the presence of capsanthin or fatty-acyl ester of capsanthin, or an extract of any natural products containing the same.

Food and/or pharmaceutical compositions according to embodiments of the present invention may be effective in preventing, inhibiting and/or treating obesity.

In the case where the food and/or pharmaceutical composition of the present invention includes free-form capsanthin, such free-form capsanthin is absorbed more easily into adipocyte and/or pre-adipocyte and hence may directly react with the same to render rapid effects.

When the food and / or pharmaceutical composition of the present invention includes esterified-form capsanthin, the ester bonds are hydrolyzed into the free-form by the enzymes as lipase or cholesterol esterase in the gastrointestinal tract(GI tract) and then absorbed into the same, hence blood level curve of the foregoing component is not rapidly altered, hence may offer more prolonged effects. In addition, the fatty-acyl ester of capsanthin is stable and generates less amounts of artifact than the free-form capsanthin.

Capsanthin is contained in the natural products such as red peppers as the chemically stable esterified-form. Therefore, even when red peppers or the likes are included in fermented foods as the Korean traditional foods like " kimchi" or "kochujang" (also referred to as "gochujang"), original anti-adipogenic activities thereof are retained. On the other hand, it was found in our laboratory that about 35% or more of anti-adipogenic capsaicin component was destroyed during 6 month-long fermentation.

Anti-adipogenic activity of capsanthin is much more excellent than that of capsaicin in respect to the lower IC₅₀-value compared to that of capsaicin and also capsanthin is devoid of local irritation or spicy taste even in contact with higher concentration.

Capsanthin or an extract of any natural products containing the same is inhibiting the differentiation of pre-adipocyte into adipocyte and also inhibiting the accumulation of fat in the adipocyte.

### BRIEF DESCRIPTIONS OF THE DRAWINGS

The above and other objects, features and other advantages of the present invention will be more clearly understood from the following detailed description taken in conjunction with the accompanying drawing, in which:

FIG. 1 shows the H-MNR result of esterified-form capsanthine;

FIG. 2 shows the H-MNR result of free-form capsanthine; and

FIG. 3 shows the HPLC-pattern of purified capsanthin and esterified-form capsanthin. Both compounds are showing chromatographically single peaks.

### DETAILED DESCRIPTION

Hereinafter, the present embodiments will be described more fully with reference to the accompanying drawing, in which exemplary embodiments are shown. As used herein, the term "and/or" includes any and all combinations of one or more of the associated listed items. Expressions such as "at least one of," when preceding a list of elements, modify the entire list of elements and do not modify the individual elements of the list.

One or more embodiments of the present invention describe a novel use of capsanthin and/or fatty-acyl esters of capsanthin to inhibit both the differentiation of pre-adipocyte to adipocyte and the accumulation of fat in adipocytes.

More particularly, one or more embodiments of the present invention are based on the findings that capsanthins contained in some natural products such as red peppers, paprika, bell peppers, etc. have anti-adipogenic activity by inhibiting both the differentiation of pre-adipocyte into adipocyte and the accumulation of fat in adipocytes, therefore, this findings provide a functional food and a pharmaceutical composition wherein capsanthins and the extract of any natural products containing the same components are included in the functional food and the pharmaceutical composition, to offer obesity prevention and/or treatment effects.

The term "functional food" is defined as a food where at least one existing ingredient has been changed and/or at least one new ingredient has been added to a food to have a new function or an improved function. For example, the change of the ingredient may be in an amount, its structure, etc. For example, the functional foods may include processed food or foods fortified with additives.

Red pepper is a traditional spicy vegetable food and more than 90% of total consumption in Korea is used to prepare traditional fermented food such as " kimchi" and "kochujang" in Korea. The present inventors have found that the intact esterified-form capsanthin was remained without any chemical alteration during fermentation of "kochujang", however, anti-adipogenic capsaicin component showed significant decomposition during the fermentation process. Based on this observation, present inventors are claiming that the anti-adipogenic activity of "kochujang" is due more to capsanthin components rather than to the capsaicin or capsaicin metabolite. Furthermore, the present inventors found that anti-adipogenic activity of fatty acyl-ester of capsanthin contained in hot pepper or "kochujang" showed highly enhanced anti-adipogenic activity when the esterified capsanthins were saponified.

The functional food and/or pharmaceutical composition of an embodiment of the present invention may include capsanthin or fatty-acyl ester of capsanthin represented by the following Formula -1 as an active ingredient:

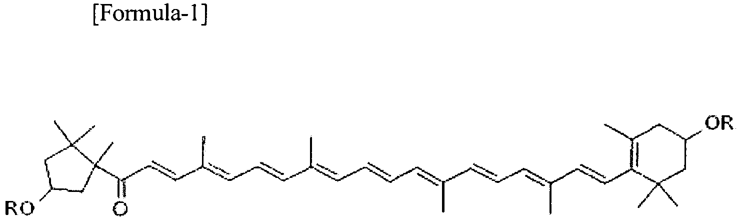

wherein R denotes hydrogen, lauroyl, linoleoyl, myristoyl or palmitoyl group

Capsanthin may be chemically synthesized or isolated from capsanthin containing natural products such as red peppers, paprika, bell peppers and the like. Alternatively, capsanthin may be obtained from fermented food stuffs such as spicy and hot red pepper sauce ("kochujang"), "kimchi", or the like.

Saponification of esterified-form capsanthin may produce free-form capsanthin together with a fatty acid mixture including, for example, 26.5% of lauric acid, 48.3% of myristic acid, 17.6% of palmitic acid and 7.5% of linoleic acid (see M. Isabel Minguez-Mosquera, Changes in carotenoid esterification during the fruit ripening of capsicum annuum Cv. Bola, J. Agric. Food Chem., 1994, 42, 640-644). When the esterified-form capsanthin is given orally to animal, the esterified-form capsanthin is converted into a tree-form capsanthin due to digestive enzymatic hydrolysis and then absorbed in the gastrointestinal (GI) tract

Methods for synthesis, separation, purification and preparation of the capsanthin and esterified-form capsanthin may not be particularly limited. For the pure isolation of capsanthin from the natural products, a customary process including finely crushing a dried natural products, extraction with a sufficient amount of organic solvent through various extracting procedure using various apparatus and subjecting the extract to solvent fractionation and then finally silica gel-column chromatography may be performed to isolate the foregoing component capsanthin in a chemically pure state from the natural products.

The functional food and/or pharmaceutical composition of an embodiment of the present invention may include capsanthins in an ester form such as fatty-acyl ester of capsanthin and/or in a free form and, optionally, other ingredients having the same or similar efficacy as that of the foregoing components. For instance, if the functional food or pharmaceutical composition includes a spicy and hot red pepper sauce extract as an active ingredient, the sauce extract substantially contains capsanthin, genistein, daidzein, capsaicin, and the like. Accordingly, since capsanthin and other active ingredients having the same or similar efficacy as that of capsanthin are included in the functional food or pharmaceutical composition, stronger anti-obesity effects may be attained.

For administration of the inventive composition, the composition may be prepared by further including at least one pharmaceutically acceptable carrier in addition to the foregoing active ingredients.

The pharmaceutically acceptable carrier may include, for example, saline, sterilized water, Ringer's solution, buffered saline, a dextrose solution, a maltodextrin solution, glycerol, ethanol, or a mixture of at least one among the foregoing materials, but is not limited thereto. Optionally, other typical additives such as an antioxidant, a buffer solution, a bacterio-static agent, etc. may be further added to the composition. Moreover, diluents, a dispersing agent, a surfactant, a binder and a lubricant may be further added to prepare formulations, i.e., injection formulation such as an aqueous solution, a suspension, an emulsion, etc., pills, capsules, granulates or tablets.

Furthermore, any conventional method suitably used in the art or a process reported in published documents, for example, "Remington's Pharmaceutical Science (Mack Publishing Company, Easton PA,18th, 1990)" may be applied to produce formulations on the basis of different diseases or various active ingredients.

The inventive composition may be used for parenteral administration (for example, intravenous (IV), subcutaneous, intraperitoneal (IP) or local application) or may be orally administered, and dosage may be determined in a broad range depending upon various parameters such as body weight, age, gender, health condition or diet of a patient, administration time, administration method, excretion rate, severity of disease, and the like. Daily dose may range from 10 to 1000 mg/kg and, for example, 10 to 100 mg/kg for an extract of any natural material (hereinafter referred to as 'natural extract') according to an embodiment of the present invention, while capsanthin may range from 0.01 to 10 mg/kg and, for example, 0.01 to 1 mg/kg. Also, it is suitable to administer the foregoing material once through several times a day.

The inventive composition may be administered alone or, otherwise, compatibly applied with surgery, radiation treatment, hormone therapy, chemotherapy and/or use of a biological response modifier, so as to inhibit and/or treat obesity.

Capsanthin or fatty-acyl ester of capsanthin, and natural extracts including the same according to an embodiment of the present invention may be added to existing functional foods in order to inhibit and/or prevent obesity. In the case where capsanthin or the extract of natural products containing the same are used as a food additive, other foods or food components may also be used in combination with the foregoing materials by conventional methods. A mixing amount of an active ingredient may be suitably determined in view of uses thereof (prevention, inhibition or remedial treatment). However, when the above active ingredient is applied to long-term medication for health and hygienic use, or for physiological control, administered amount may be less than the foregoing range. Since capsanthin and any extract of natural products containing the same have no problem in an aspect of biological safety, an active ingredient may be used in an amount of more than the foregoing defined range.

Kinds and/or types of foodstuffs are not particularly limited. Examples of foodstuffs possibly containing the materials described above may include meat, sausages, bread, chocolate, candy, snacks, confections, pizza, ramen noodles, other noodles, gum, diary products including ice cream, various soups, beverages, teas, drinks and alcohol drinks, vitamin complex, etc., and substantially include overall functional foods commonly known in the art.

A health drink composition according to an embodiment of the present invention may further include variety flavors or natural carbohydrates as additional ingredients, like general beverages.

Such natural carbohydrates may include, for example: monosaccharide such as glucose, fructose, etc.; disaccharides such as maltose, sucrose, etc.; polysaccharides such as dextrin, cyclodextrin, etc.; sugar alcohol such as xylitol, sorbitol, erythritol, etc., and so forth. A sweetening agent may include, for example: natural sweeteners such as thaumatin, stevia extract, etc.; or synthetic sweeteners such as saccharin, aspartame, etc.

Content of the natural carbohydrate may range about 0.01 to 0.04g and, for example, 0.02 to 0.03g to 100 ml of the inventive composition.

Other than the foregoing materials, the inventive composition according to an embodiment of the present invention may further include different nutrients, vitamins, electrolytes, flavors, coloring agents, pectic acid and salts thereof, alginic acid and salts thereof, organic acid, protective colloidal thickeners, pH modifiers, stabilizers, preservatives, glycerin, alcohol, a carbonating agent used for carbonated drinks (so called 'sparkling drinks'), or the like. According to an embodiment of the present invention, the inventive composition may additionally include natural fruit juice, fruit juice beverage and/or fruit puree used for manufacturing vegetable drinks.

The components described above may be used independently (alone) or as a mixture thereof. Ratios of such additives are not critical but may be determined within a range of 0.01 to 0.1 parts by weight to 100 parts by weight of the inventive composition.

Meanwhile, an embodiment of the present invention may provide a method of inhibiting the differentiation of adipocyte and accumulation of triglycerides in adipocytes wherein capsanthin and/or fatty-acyl ester of capsanthin represented by Formula 1 or a natural extract containing the same react with adipocyte and/or pre-adipocyte thereof.

Hereinafter, embodiments of the present invention will be described in detail to understand more concretely the present invention with reference to examples and comparative examples. However, it will be apparent to those skilled in the art that such embodiments are provided for illustrative purposes and do not limit subject matters to be protected as defined by the appended claims.

**EXAMPLE**

**Example 1: Preparation of ethyl acetate extract of red pepper**

50L of ethyl acetate was mixed with 10kg of red pepper powder, and the mixture was refluxed for 3 hours in a boiling water bath. The extractions were repeated 3 times. After cooling, the treated mixture was filtered and the filtrate was vacuum concentrated to give 1.53kg of a red pepper extract.

**Example 2: Preparation of paprika extract in ethyl acetate**

50L of ethyl acetate was mixed with 10kg of dried paprika powder and the mixture was refluxed for 3 hours in a boiling water bath. The extractions were repeated 3 times. After cooling, the treated mixture was filtered and the filtrate was vacuum concentrated to give 825.2g of a paprika extract.

**Example 3: Preparation of bell pepper extract in ethyl acetate**

50L of ethyl acetate was mixed with 10kg of dried bell pepper powder and the mixture was refluxed for 3 hours in a boiling water bath. The extractions were repeated 3 times. After cooling, the treated mixture was filtered and the filtrate was concentrated to give 784.3g of a bell pepper extract.

**Example 4: Preparation of spicy and hot red pepper sauce extract in ethyl acetate**

10kg of spicy and hot red pepper sauce (traditional Korean food name; "kochujang") fermented over 3 months was lyophilized to remove water content, thus preparing 7kg of freeze dried product. Then, 50L of ethyl acetate was mixed into the above product and the mixture was refluxed for 3 hours in a boiling water bath. The extractions were repeated 3 times. After cooling, the treated mixture was filtered and the filtrate was vacuum concentrated to give 1.28kg of a spicy and hot red pepper sauce extract.

**Example 5: Preparation of capsaicin-free capsanthin-containing extract from red pepper extract in ethyl acetate**

1.5kg of a red pepper extract was dissolved in 7L of hexane, and mixed with 7L of 0.1N-alkaline solution in separating funnel and shaked very vigorously in separating funnel to remove capsaicin by alkali washing. Alkali-washing processes were repeated 5- times. The complete removal of capsaicin could be confirmed by HPLC analysis on hexane-layer.

HPLC-conditions for capsaicin analysis: Column; C8-reverse phase column (Agilent Eclipse XDB-C8, 150x4.6 mm, 5µm), Developing solvent; methanol : water = 4:6, Flow rate; 1 ml/min, Retention time of capsaicin; 18.59 minutes, Detection; UV absorption at 230 nm].

Hexane layer was washed with 0.5N-HCl to remove excessive alkali and resulting hexane layer was washed with saturated sodium-bicarbonate solution to remove excessive hydrogen chloride Hexane layer containing capsanthin was dehydrated by mixing enough amount of anhydrous sodium sulfate. Dehydrated hexane-layer containing capsanthin was filtered and vacuum evaporated to give 1.27kg of capsaicin-free capsanthin containing extract.

**Example 6: Preparation of capsiate-free capsanthin-containing extract from paprika extract in ethyl acetate**

Ethyl acetate extract of Paprika(800g) was treated by the same procedure as described in Example 5 to give 536.3g of capsiate-free capsanthin containing extract.

**Example 7: Preparation of capsiate-free capsanthin-containing extract from bell pepper extract in ethyl acetate**

Ethyl acetate extract of bell pepper (700g) was treated by the same procedure as described in Example 5 to give 487.7g of capsiate-free capsanthin containing extract.

**Example 8: Preparation of capsaicin-free capsanthin-containing extract from spicy and hot red pepper sauce extract in ethyl acetate**

Ethyl acetate extract of spicy and hot red pepper sauce (1.2kg) was treated by the same procedure as described in Example 5 to give 753.4g of capsaicin-free capsanthin containing extract.

**Example 9: Pure Isolation of esterified-form capsanthin from capsanthin-containing extract**

1kg of oily-liquid capsaicin-free capsanthin fraction (of Example 5) was dissolved in 30L absolute ethanol and mixed well with catalytic amount of sodium-methoxide and stirred for a while at room temperature to fulfill trans-esterification reaction. After the completion of trans-esterification, sodium-methoxide catalyst was deactivated by the addition of equimolar amount of acetic-acid and vacuum-evaporated to obtain 1.03kg dark-red oily mixture of fatty-acyl ester of capsanthin, fatty-acid ethyl-esters and glycerine. (Triglyceride will be transesterified to give ethyl-esters of fatty acids, however, fatty-acyl ester of capsanthin will not be attacked in this trans-esterification condition, Ref; T. Philip, W. W. Nawar and F. J. Francis, The nature of fatty acids and capsanthin esters in paprika, Journal of Food Science., 1971, 36, 98-100)

A silica-gel(2kg) column was constructed with hexane, and loaded with hexane solution of the transesterified product(1kg) containing the mixture of fatty-acyl ester of capsanthin and fatty acid ethyl-ester and eluted exhaustively with hexane to remove fatty-acid ethyl-ester completely and then the elution solvent was changed from hexane to the mixed solvent of hexane : ethyl-acetate = 20 : 1 to give 8.75g of the fatty-acyl ester of capsanthin as a single spot on TLC.

8.75g of the fatty-acyl ester of capsanthin was dissolved in hexane and 1.54g of dark red amorphous powder was obtained at 4°C.

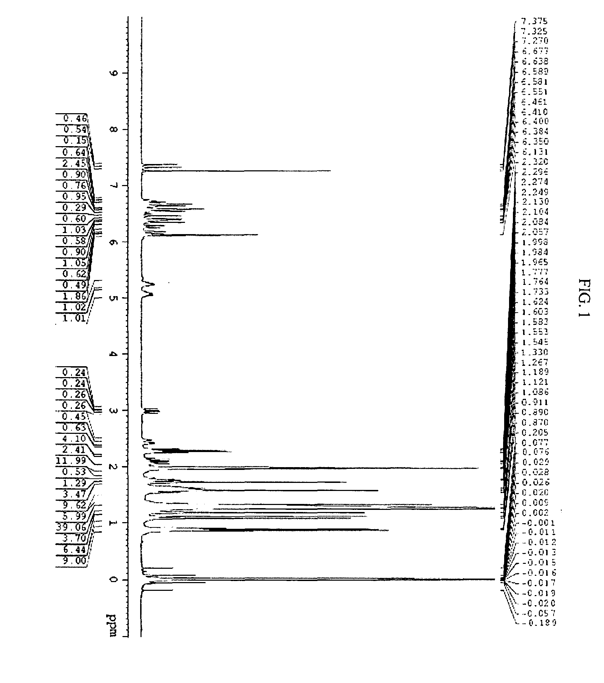

PMR-spectra of the dark red amorphous powder was obtained in CDCl₃-solvent on 500MHz NMR and its spectra assignment confirmed fatty-acyl ester of capsanthin (FIG. 1).

**Example 10: Isolation of free-form capsanthin by saponification of esterified-form form capsanthin**

Esterified-form form capsanthin (1.0g) in 5ml ethanol was saponified by warming in 65°C-water bath during one hour with 20 ml 0.1 N-NaOH in ethanol under nitrogen replacement. After cooling, the reaction mixture was vacuum evaporated to give the mixed residue of dark red capsanthin and sodium salts of fatty acids. The dark red residue was extracted with 20ml ethyl ether three times to remove the fatty acid sodium salt and excessive alkali as the insoluble residue. The ethyl ether solution of free capsanthin was evaporated to give 420mg free form capsanthin together with some carotenoid impurities as an amorphous residue.

280mg of dark red crystalline powder (needle) was obtained in methylene chloride.

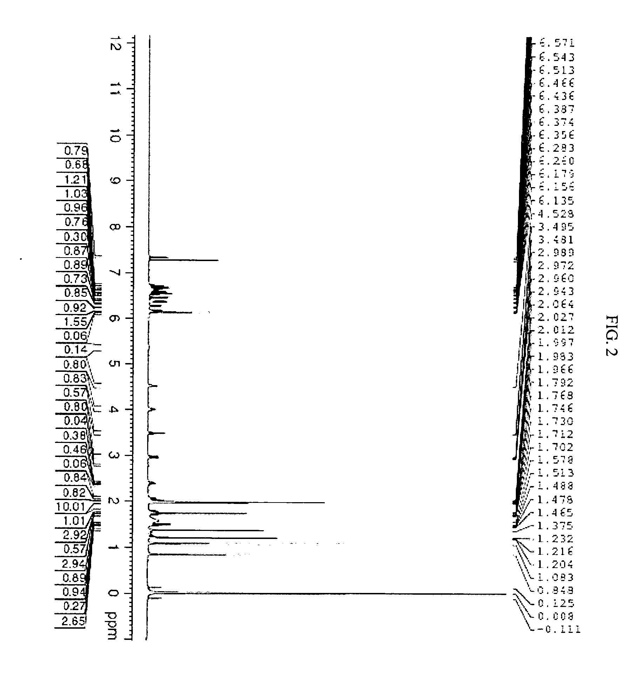

PMR-spectra of the dark red crystalline powder (needle) was obtained in CDCl₃-solvent on 500MHz NMR and its spectra assignment confirmed free-form capsanthin (FIG. 2).

Proton- NMR spectra of the free capsanthin is completely superimposable with the PMR-data appeared in the reference( Refer to; Ruttimann, A. et al., Helv. Chim. Acta 1983, vol.66, Fasc.7, p1939-1960)

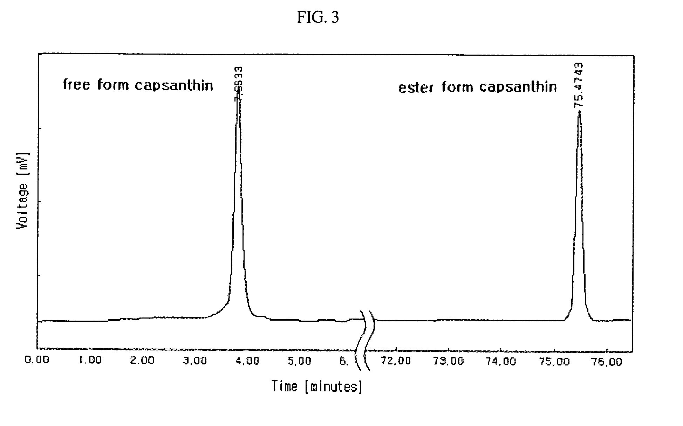

Free-form capsanthin and esterified-form capsanthin were confirmed chromatographically single peak through HPLC(FIG.3).

HPLC analysis conditions: Column; ODS-120A column (TOSOH, tsk-gel, 150x4.6mm, 5µm), developing solvent; acetonitrile : 2-propanol : ethyl acetate (8:1:1), flow rate of developing solvent; 0.8 ml/min, retention time of free-form capsanthin; 7.60 minutes, retention time of free-form capsanthin; 75.47 minutes, detection; UV absorption at 480nm)

**Example 11: Confirmation of inhibitory activities of capsanthin on adipocyte differentiation and on triglycerides accumulation in adipocyte by using 3T3-L1 CELL**

3T3-L1 mouse embryonic fibroblast was cultured according to Singh's method (Singh R, Testosterone inhibits adipogenic differentiation in 3T3-L1 cells: Nuclear translocation of androgen receptor complex with β-catenin and T-cell factor 4 may bypass canonical WNT signaling to down-regulate adipogenic transcription factors, Endocrinology, 2006, 147, 141 to 154).

3T3-L1 cells supplied by Korean cell line bank were cultured on a Dulbecco's modified Eagle's medium (DMEM) medium that includes 10% bovine calf serum and 1% penicillin-streptomycin mixture. When they reached confluence, culturing was continued for further 2 to 3 days. Thereafter, the medium was replaced by a fresh DMEM medium containing 10% fetal bovine serum, 1.7µM insulin, 0.5mM 3-isobutyl-1-methylxanthine (IBMX) and 1µM dexamethasone and culturing was continued for 8 days. For the evaluation of inhibitory activities of capsanthin on adipocyte differentiation and on triglycerides accumulation in adipocyte, a positive control experiments were conducted on capsaicin (sigma) and/or genistein (sigma).

In this regard, capsaicin and genistein has already been known as potent anti-adipogenic substance, which are contained in red peppers and fermented soybean bricks (or malt; so-called 'meju'), respectively.

**1) Inhibition test for capsanthin on adipocyte differentiation**

Mouse embryonic 3T3-L1 cells were plated in 12-well culture plate with DMEM containing 10% BCS(Bovine calf serum) and cultured for the proliferation of cells under 5% CO₂ atmosphere at 37°C until confluent phase is appeared. Continued culture for additional 2 days will ensure the clonal expansion. Culture media is replaced with differentiation medium consisting of DMEM, 10% FBS, 1.7 uM insulin, 0.5 mM IBMX, 1 uM dexamethasone and varying amount of capsanthin in DMSO (final concentration of DMSO in the differentiation media were kept under 0.1%) and cultured additional 3 days for the induction of cell differentiation Culture media was removed and washed with PBS and the cells were fixed on the well surface by incubation with 10% formaldehyde for one hour and then washed with PBS. The differentiate cells were stained with Oil Red O solution for 2 hours. The number of adipocyte stained red was counted with microscopical observation.

Fifty percent inhibition concentrations (IC₅₀-values) of capsanthins and other reference compounds of adipocyte differentiation were determined by microscopical counting of Oil Red O stained cells as followings; IC₅₀-value for free-form capsanthin as 4.49 uM, for esterified-form capsanthin as 75.43 uM, for capsaicin 3.39 uM, for genistein as 28.10 uM and for fucoxanthin as 79.11 uM

**2) The assay of the inhibitory activity of capsanthin on triglycerides accumulation in adipocyte**

Negal's Oil red O staining procedure (Negrel R, Culture of adipose precursor cell and cells of clonal lines from animal white adipose tissue, in adipose tissue protocols. G. Ailhaud(ed). Humana press, inc., Totowa, NJ, 2001, 225-237) was adopted to assay the quantities of triglyceides accumulated in adipocytes. The pre-adipocytes (3T3-L1 cell) were cultured in 12-well culture plate for additional two days to induce clonal expansion after the confluent phase. The culture media was replaced with differentiation media which is containing various concentration of capsanthin solution in DMSO (final concentration of DMSO in the differentiation media were kept under 0.1 %) and cultured for additional 8 days to induce lipid accumulation. The culture media was removed by aspiration and washed with PBS. The cells were fixed on the surface of culture well by incubation for one hour with 500 ul of 10% formaldehyde solution. After removal of formaldehyde the cells were washed with PBS and then stained by incubation with Oil Red O for 2 hours. After two removal of Oil Red O solution by aspiration the cells were washed with PBS and then Oil Red O in adipocyte was extracted by incubation 10 minutes with 1ml isopropyl-alcohol. Oil Red O contents in isopropyl-alcohol were assayed by 510nm absorption on ELISA reader.

Fifty percent inhibition concentrations (IC₅₀-values) of capsanthins and other reference compounds on triglycerides accumulation in adipocyte were determined by microscopical counting of Oil Red O stained cells as followings; IC₅₀-value for free-form capsanthin as 1.31 uM, for esterified-form capsanthin as 55.24 uM, for capsaicin 32.65 uM, for genistein as 58.74 uM and for fucoxanthin as 54.81 uM.

**Example 12: gastro-intestinal absorption of capsanthin**

**Experiment No-1 ;** Male Sprague-Dawley albino rats were used for the analysis of gastro-intestinal absorption of capsanthins. Animals were accommodated in a constant humidity of 50 ( ± 5)% and constant temperature of 25 (±1) °C under shading control of light and dark with 12 hours intervals. Feeds and water supplies were provided at libidium.

Free-form capsanthin(15mg/kg body wt) in soy-bean oil was orally administered to group-1 animals and then 4ml time-course blood samples were collected from the animals to EDTA tubes and centrifuged (3,000 rpm, 10 min) to obtain 1ml plasma samples. To the plasma samples 1ml of 1%-BHT in ethanol and 2ml hexane were mixed, shaked vigorously and centrifuged for 5 minutes at 2,000 rpm to obtain hexane layer for the HPLC-analysis under the HPLC-condition appears in Example 10. (M. A. Grashorn, Quantification of carotenoids in chicken plasma after feeding free of esterified lutein and capsanthin using high-performance liquid chromatography and liquid chromatography-mass spectrometry analysis, Poultry science, 2003, 82, 395-401, Hideo E, Carotenoids in human blood plasma after ingesting paprika juice)

Fatty-acyl ester of capsanthin was orally administered to group-2 animals equimolar dosage bases with that of free-form capsanthin. The time course collection of blood and further treatments were conducted as same way with that of group-1 experiments [Ref. to 0086]

**Experiment No-2** ; Capsanthin (free-form) and esterified-form capsanthin were administered to animals as the same way in Experiment No-1 except that once/per-day for 7 days and time course blood-samples were collected at 7th- day.

As the results of blood analysis by HPLC, it was found that exclusively free-form capsanthin was appeared in the blood and esterified-form was not detected irrespective of orally administered molecular species of capsanthins. These results are suggesting that free-form capsanthin only could be absorbed in the gastro-intestinal tract after enzymatic hydrolysis of esterified-form capsanthin by some digestive enzymes as lipase or cholesterol-esterase.

As appeared in Table 1 and Table 2, capsanthin concentration in the blood plasma was rapidly elevating without any lag-period, when the free-form capsanthin was administered orally, however, the capsanthin concentration curve shows some lag-period and blood level was much lower, when the esterified-form capsanthin was administered orally, compared to the free-form capsanthin administration

**[TABLE 1]**

| Blood collection time after single administration | Content of capsanthin in blood (mV×sec) | |
|---|---|---|
| | Administration of free-capsanthin | Administration of esterified-form capsanthin |
| After 1 hour | Not detected | 4.18 |
| After 2 hours | 132.53 | 25.36 |

**[TABLE 2]**

| Blood collection time after the final administration among administration for 7 days | Content of capsanthin in blood (mV×sec) | |
|---|---|---|
| | Administration of tree-form capsanthin | Administration of esterified-form capsanthin |
| After 0 hour | Not detected | Not detected |
| After 0.5 hour | 8.11 | Not detected |
| After 1 hour | 100.13 | 47.65 |
| After 2 hours | 147.24 | 67.75 |
| After 4 hours | 224.17 | 87.47 |

**Example 13: Preparation of capsanthin-containing drink**

After suitably adding small amounts of water, lecithin extracted from beans and any proper additive, *i.e*., a condiment, to capsanthin as an anti-adipogenic agent, the mixture was subjected to homogenization at a high speed to form an emulsion. According to a common method for manufacturing beverages, the emulsion product was packed into brown bottles as a single dose, followed by nitrogen replacement and heating for sterilization at 105°C for 1 hour, thereby manufacturing drink products.

**Example 14: Preparation of capsanthin-containing capsule**

After mixing capsanthin as an anti-adipogenic agent with a potato starch or the like to prepare a homogeneous diluted powder, capsules were prepared according to any conventional process.

**Example 15: Preparation of capsanthin-containing tablet**

After homogenously mixing capsanthin as an anti-adipogenic agent with proper amounts of diluents and other excipients used for tablet formulation, the mixture was subjected to tablet pressing according to any conventional process, thereby manufacturing tablets.

Although embodiments of the present invention have been described above in conjunction with the accompanying examples and experimental examples, those skilled in the art will appreciate that various modifications and alterations are possible without departing from the scope and spirit of the invention, based on the foregoing description and the appended claims.

## Claims

1. A product comprising a functional food or a pharmaceutical product for preventing or inhibiting obesity, the product comprising a composition comprised of at least one of capsanthin, fatty-acyl ester of capsanthin represented by Formula 1, and an extract of natural material containing the capsanthin, said fatty-acyl ester of capsanthin or the combination thereof: 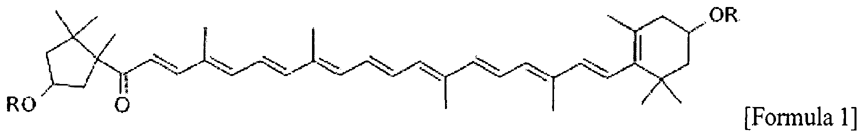 wherein R denotes hydrogen, a lauroyl group, a linoleoyl group, a myristoyl group or a palmitoyl group.

2. The product according to claim 1, wherein the product comprises the functional food.

3. The product according to claim 1, wherein the product comprises the pharmaceutical product.

4. The product according to claim 1, wherein the composition comprises the extract of natural material, and the natural material is at least one selected from a group consisting of red pepper, paprika and bell pepper.

5. The product according to claim 1, wherein the composition comprises the extract of natural material comprising the capsanthin, and the capsanthin included in the natural material is an esterified-form.

6. The product according to claim 1, wherein the product is beverages or confectionary products.

7. The product according to claim 1, wherein the product is grains, cereals, breads, or noodles.

8. The product according to claim 1, wherein the product is a pharmaceutical product further comprising a pharmaceutically acceptable carrier.

9. The product according to claim 2, wherein the composition is an immediate-release type or controlled release type formulation.

10. The product according to claim 1, wherein the capsanthin is isolated from kochujang or Kimchi.

11. Use of a composition comprising at least one of capsanthin and fatty-acyl ester of capsanthin by Formula 1, and an extract of natural material containing the capsanthin, said fatty-acyl ester of capsanthin or the combination thereof, for the manufacture of a medicament for the treatment or prevention of obesity: 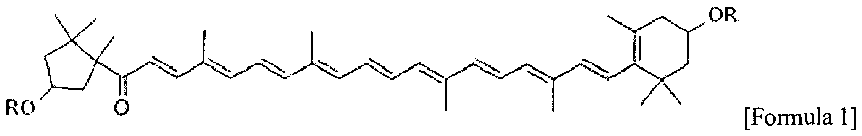 wherein R denotes hydrogen, a lauroyl group, a linoleoyl group, a myristoyl group or a palmitoyl group.

12. The use of claim 11, wherein capsanthin contained in the natural material is an esterified-form.

13. The use of claim 11, wherein the composition is a pharmaceutical composition further comprised of a pharmaceutically acceptable carrier.

14. The use of claim 13, wherein the pharmaceutical composition consists essentially of said at least one of capsanthin, its ester derivative and the extract of natural material, and the pharmaceutically acceptable carrier.

15. The use of claim 11, wherein the natural material is at least one selected from a group consisting of red pepper, paprika and bell pepper.
